**Europäisches Patentamt**

**European Patent Office**

**Office européen des brevets**

(19)

(11) Veröffentlichungsnummer: **0 137 302**
**A2**

(12)

# EUROPÄISCHE PATENTANMELDUNG

(21) Anmeldenummer: **84110550.5**

(22) Anmeldetag: **05.09.84**

(51) Int. Cl.⁴: **A 61 K 33/14**
A 61 K 9/06, A 61 K 45/06
//(A61K33/14, 31:70, 31:765)

(30) Priorität: **12.09.83 FR 8314445**

(43) Veröffentlichungstag der Anmeldung:
**17.04.85 Patentblatt 85/16**

(84) Benannte Vertragsstaaten:
**BE CH DE FR GB IT LI LU NL SE**

(71) Anmelder: **Anasco GmbH**
**Aarstrasse 1**
**D-6200 Wiesbaden(DE)**

(72) Erfinder: **Gros, Pierre, Dr.**
**9 villa Brune**
**F-75014 Paris(FR)**

(74) Vertreter: **Sommer, Fritz, Dr. et al,**
**Boehringer Ingelheim International GmbH ZA Patente**
**Postfach 200**
**D-6507 Ingelheim/Rhein(DE)**

(54) Mittel zur Verhinderung des Schnarchens.

(57) Die vorliegende Erfindung betrifft ein Mittel zur Verhinderung des Schnarchens. Dieses Mittel ist zur Anwendung an den rhino-pharyngitischen Schleimhäuten bestimmt und besteht aus einer gefäßschützenden Verbindung, einer isotonischen Salzlösung, einem oberflächenaktiven Mittel, das mit den rhino-pharyngitischen Schleimhäuten verträglich ist, und gegebenenfalls aus anderen schleimhautverträglichen Zusätzen.

EP 0 137 302 A2

Croydon Printing Company Ltd.

Case 11/011

0137302

Dr. So./Dt

ANASCO Gesellschaft mit beschränkter Haftung,
Aarstrasse 1, 6200 Wiesbaden

Mittel zur Verhinderung des Schnarchens

Die vorliegende Anmeldung betrifft ein Mittel zur Verhinderung des
Schnarchens. Das Schnarchen ist ein lautstarkes Phänomen, das auf
einer rasselnden Atmung beruht, die bei verschiedenen Personen während ihres Schlafes auftritt. Ohne die Erfindung auf eine erläuternde Theorie zu beschränken, wird der Ansicht Ausdruck gegeben, dass
das lautstarke Phänomen des Schnarchens durch ein aerodynamisches
und akustisches Phänomen hervorgerufen wird, welches durch die Austrocknung der rhino-pharyngitischen Schleimhäute entsteht, und dass
durch einen Befeuchtung dieser Schleimhäute die Verhinderung des
Schnarchens ermöglicht wird.

Es war festgestellt worden, dass das Schnarchen bei Schnarchern verhindert werden kann, indem man auf die rhino-pharyngitischen
Schleimhäute ein geeignetes Mittel aufbringt, um die genannten
Schleimhäute in befeuchtetem Zustand zu halten. Es wurde nunmehr
festgestellt, dass die Austrocknung dieser Schleimhäute auch die
Brüchigkeit der Kapillaren des darunter liegenden Gewebes erhöht;
diese Brüchigkeit kann dadurch ausgeglichen werden, dass auf die
Schleimhäute ein gefässchützendes Mittel appliziert wird.

Die Erfindung betrifft also ein Mittel zur Verhinderung des Schnarchens, welches folgende Bestandteile enthält:

a.     ein gefässchützendes Mittel

b.     eine mit dem Blutserum isotonische Salzlösung, die
       vorzugsweise auf einen pH von 6,8 gepuffert ist

c.     ein mit den rhino-pharyngitischen Schleimhäuten verträgliches oberflächenaktives Mittel und

d.     gegebenenfalls weitere Zusätze, die mit den genannten
       Schleimhäuten verträglich sind.

Das neue Mittel enthält eine gefässchützende bzw. gefässaktive Substanz, vorzugsweise aus der Gruppe der Flavonoide. Als nichteinschränkendes Beispiel für eine geeignete gefässchützende Substanz
kann Hesperidinmethylchalkon genannt werden. Diese Substanz wirkt
gefässchützend und verringert daher die bei der Austrocknung der Nasenschleimhaut häufig auftretende Blutung, insbesondere bei fortgeschrittenem Alter.

Im erfindungsgemässen Mittel wird die Befeuchtung der Schleimhäute
durch eine Salzlösung erreicht, die mit dem Blutserum isotonisch
ist. Diese Salzlösung in entmineralisiertem Wasser kann beispielsweise aus Natriumchlorid und/oder Calciumchlorid und/oder Kaliumchlorid bestehen. Für die Pufferung auf pH 6,8 der für eine intranasale Anwendung besonders günstig ist, können übliche physiologisch
verträgliche Puffersysteme, wie Mc.Ilvaine Puffer oder Sörensen-Puffer verwendet werden.

Die oberflächenaktive Substanz, die in dem neuen Mittel enthalten
ist, spielt eine wichtige Rolle, den Kontakt der isotonischen Salzlösung mit den rhino-pharyngitischen Schleimhäuten zu begünstigen
und aufrechtzuerhalten. Diese oberflächenaktive Substanz, muss mit
den genannten Schleimhäuten verträglich sein. Als typische Beispiel
von geeigneten oberflächenaktiven Substanzen kann man die Polyhydroxyäthylsorbitanester erwähnen, also die Ester des Sorbitans, an
deren freien Hydroxylgruppen eine bestimmte Anzahl von Ethylenoxidmolekülen ankondensiert ist. Die gebräuchlichsten dieser Ester sind
diejenigen, an welche 20 Mole Ethylenoxid ankondensiert sind. Ein

0137302

besonders bevorzugtes Produkt ist Polysorbate 80, welches ein nichtionisches oberflächenaktives Mittel ist, das aus einem Monooleinsäureester des Sorbitans besteht, an welchen etwa 20 Mole von Ethylenoxid ankondensiert sind. Dieses Produkt ist in den Pharmakopöen
erwähnt, beispielsweise Eur. Pharm. III, S. 347 (1975). Es soll in
dem neuen Mittel vorzugsweise in einer Menge von 5,0 g bis 10,0 g
pro 1 Liter enthalten sein. Diese nähere Beschreibung der oberflächenaktiven Mittel soll jedoch die Erfindung in keiner Weise einschränken.

Das neue Mittel kann gleichfalls auch andere schleimhautverträgliche
Zusätze enthalten, beispielsweise Konservierungsmittel, Chelatbildner, die Schleimhaut geschmeidig machende Stoffe usw., zusammenfassend alle Substanzen, die im allgemeinen in Präparaten für die nasale Anwendung gebräuchlich sind. Beispielsweise können, ohne wiederum die Erfindung zu beschränken, Ethylalkohol oder Benzalkoniumchlorid als Konservierungsmittel genannt werden; Dinatriumedetat
(Dinatriumsalz der Ethylendiamintetraessigsäure) kann als Chelatbildner verwendet werden; Glyzerin und Panthenol sind Substanzen,
die, wie zahlreiche weitere Diglykole, als die Schleimhaut geschmeidig machende Mittel dienen.

Die Zusammensetzung der neuen Mittel kann in einem beschränkten Rahmen variiert werden, beispielsweise können die isotonischen Salze in
einer Menge von 8,0 bis 9,5 g pro 1000 ml enthalten sein, die oberflächenaktiven Stoffe in einer Menge von 5,0 bis 12,0 g; von der erfindungsgemässen gefässchützenden Substanz werden 4,0 bis 10,0 g
eingesetzt, von der die Schleimhaut geschmeidig machenden Substanz
zwischen 4,0 und 8,0 g, von dem Konservierungsmittel 0,1 bis 0,5 g,
von dem Chelatbildner 0,2 bis 1 g; jeweils auf 1000 ml.

Es waren zwar bereits ähnliche Mittel zur Bekämpfung des Schnarchens
bekannt, beispielsweise aus der europäischen Patentanmeldung
81 109 807.3. Diese bekannten Mittel helfen jedoch nicht zuverlässig
bei hartnäckigem Schnarchen, insbesondere nicht nach dem Genuss von grösseren Mengen Alkohol. Durch den erfindungsgemässen Zusatz eines
gefässchützenden Mittels können Blutungen, die sonst häufig, insbesondere bei älteren Personen, auftreten, mit Sicherheit verhindert
werden.

Durch Zusatz einer gepufferten isotonischen Salzlösung an Stelle von
reinem Natriumchlorid kann der pH-Wert, der bei der bekannten Lösung
häufig unter 5 liegt, auf 6,8 gehalten werden, der für die intranasale Anwendung optimal ist, nämlich optimal für die Zilienbewegung
des Flimmerepithels.

Die nachstehende Formulierung soll die Erfindung näher erläutern,
ohne sie zu beschränken:

| | |
|---|---|
| Natriumchlorid | 9,5 g |
| Polysorbate 80 | 12,0 g |
| Hesperidinmethylchalkon | 8,0 g |
| Glyzerin | 3,0 g |
| Panthenol | 3,0 g |
| Benzalkoniumchlorid | 0,1 g |
| Dinatriumedetat | 0,3 g |
| entmineralisiertes Wasser, auf pH 6,8 mit Mc.Ilvaine Lösung, eingestellt | ad 1000 ml |

In diesem Beispiel sind Glyzerin, Panthenol, Benzalkoniumchlorid und
Dinatriumedetat fakultative Zusätze. Mc Ilvaine Lösung besteht aus
22,75% 0,1 molarer Citronensäurelösung und 77,25% 0,2 molarer Dinatriumphosphatlösung.

Das erfindungsgemässe Mittel wird auf die Nasen- und Rachenschleimhäute einer mit Schnarchen belasteten Person appliziert. Zu diesem
Zwecke genügt ist, umgefähr 0,5 bis 10 ml, vorzugsweise 1 ml, in jedes Nasenloch mit Hilfe eines geeigneten Geräts einzuführen. Zu diesem Zwecke können die üblichen im Handel befindlichen Geräte, wie
Aerosolgerät, ein Zerstäuber oder eine Spülpipette verwendet werden.
Die Anwendung kann selbstverständlich während der Nacht wiederholt
werden. Auch bei langzeitiger Anwendung haben sich bisher keinerlei
Unverträglichkeitserscheinungen gezeigt.

Patentansprüche

1. Mittel zur Verhinderung des Schnarchens zur Anwendung auf den rhino-pharyngitischen Schleimhäuten, bestehend aus einer gefässchützenden Substanz, einer Salzlösung, die mit dem Blutserum isotonisch und verträglich und vorzugsweise auf einen pH von 6,8 gepuffert ist, aus einer mit den rhino-pharyngitischen Schleimhäuten verträglichen oberflächenaktiven Substanz und gegebenenfalls aus weiteren schleimhautverträglichen Zusätzen.

2. Mittel gemäss Anspruch 1, dadurch gekennzeichnet, dass die gefässchützende Substanz ein Flavonoid ist.

3. Mittel gemäss den Ansprüchen 1 und 2, dadurch gekennzeichnet, dass die gefässchützende Substanz Hesperidinmethylchalkon ist.

4. Mittel gemäss dem Anspruch 1, dadurch gekennzeichnet, dass die oberflächenaktive Substanz ein Ester des Polyhydroxyethylsorbitans ist.

5. Mittel gemäss den Ansprüchen 1 und 4, dadurch gekennzeichnet, dass die oberflächenaktive Substanz aus einem Monooleinsäureester des Sorbitans besteht, an den umgefähr 20 Moleküle Ethylenoxid ankondensiert sind.

6. Mittel gemäss den Ansprüchen 1 bis 5, dadurch gekennzeichnet, dass es als weitere fakultative Zusätze ein Konservierungsmittel, ein Chelatbildner und eine die Schleimhaut geschmeidig machende Substanz enthält.

7. Mittel gemäss den Ansprüchen 1 bis 6, dadurch gekennzeichnet, dass es

4,0 g bis 10 g eines Flavonoids,
8,0 g bis 9,5 g eines Salzes, das eine isotonische Salzlösung ergibt,
5,0 g bis 12 g eines oberflächenaktiven Mittels,
4,0 g bis 8,0 g einer die Schleimhaut geschmeidig machenden Substanz,
0,1 g bis 0,5 g eines Konservierungsmittels,
0,2 bis 1,0 g eines Chelatbildners
pro 1000 ml entmineralisierten Wassers, das mit einer physiologisch verträglichen Pufferlösung auf pH 6,8 gepuffert ist,

enthält.

8. Mittel gemäss den Ansprüchen 1 - 7, dadurch gekennzeichnet, dass die physiologisch verträgliche Pufferlösung die Mc.Ilvaine-Lösung ist.

9. Verfahren zur Verhinderung des Schnarchens, dadurch gekennzeichnet, dass ein Mittel gemäss den Ansprüchen 1 bis 7 auf die rhinopharyngitischen Schleimhäute aufgebracht wird.